(19) Europäisches Patentamt
European Patent Office
Office européen des brevets

(11) **EP 2 133 026 B1**

(12) **EUROPEAN PATENT SPECIFICATION**

(45) Date of publication and mention
of the grant of the patent:
**22.05.2013  Bulletin 2013/21**

(21) Application number: **08751593.8**

(22) Date of filing: **22.04.2008**

(51) Int Cl.:
***A61B 8/00*** *(2006.01)*    *A61B 5/0456* *(2006.01)*

(86) International application number:
**PCT/JP2008/001049**

(87) International publication number:
**WO 2008/132835 (06.11.2008 Gazette 2008/45)**

(54) **ULTRASONOGRAPHIC DEVICE**

ULTRASCHALLGERÄT

DISPOSITIF D'ÉCHOGRAPHIE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **24.04.2007  JP 2007113731**

(43) Date of publication of application:
**16.12.2009  Bulletin 2009/51**

(73) Proprietor: **Panasonic Corporation
Kadoma-shi
Osaka 571-8501 (JP)**

(72) Inventors:
• **FUKUKITA, Hiroshi
Shiromi 2-chome, Chuo-ku,
Osaka-shi, Osaka 540-6207 (JP)**

• **FUKUMOTO, Takenori
Shiromi 2-chome, Chuo-ku,
Osaka-shi, Osaka 540-6207 (JP)**

(74) Representative: **Kügele, Bernhard et al
Novagraaf International SA
Chemin de l'Echo 3
1213 Onex (CH)**

(56) References cited:
**EP-A1- 1 582 149      GB-A- 2 092 746
JP-A- 10 328 188       JP-A- 2000 232 978
JP-A- 2001 269 344     JP-A- 2004 000 613
JP-A- 2005 111 131     JP-A- 2005 111 131
US-A- 5 964 706        US-A1- 2003 018 259**

**Description**

TECHNICAL FIELD

The present invention relates to an ultrasonographic device using complementary coding transmission and reception. An ultrasonographic device corresponding to the preamble of present claim 1 is disclosed by the document JP 2005 111131.

BACKGROUND ART

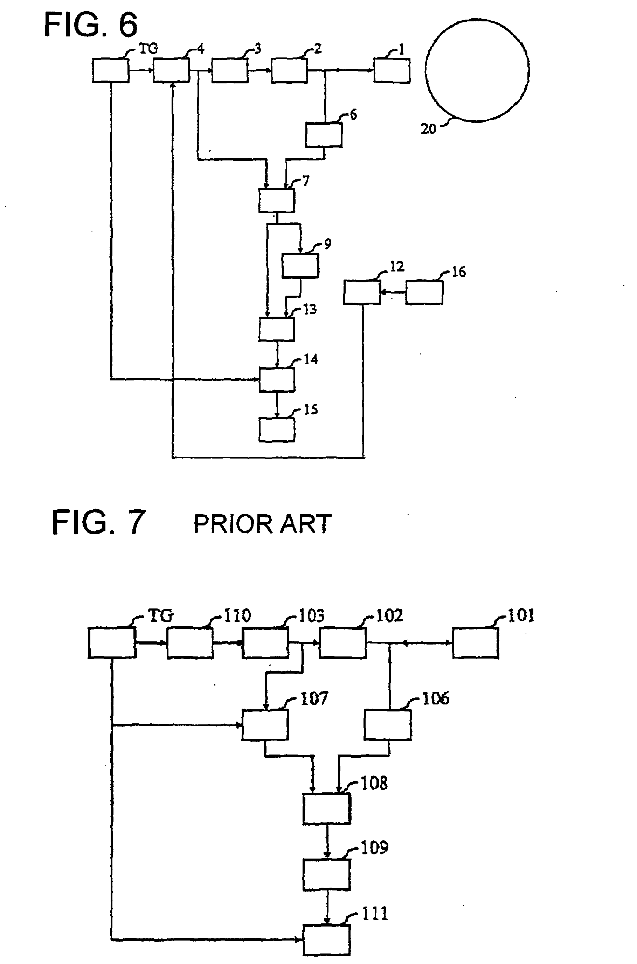

A conventional ultrasonographic device is configured such that as shown in FIG. 7, an output of a coded waveform generation unit 103 is supplied to a transmission unit 102 which drives a prove 101 in accordance with an output of a complementary code generator 110; a reference waveform storage unit 107 stores the output of the coded waveform generation unit 103; a reception amplification unit 106 amplifies a received signal from the prove 101; a correlator 108 performs a correlation calculation between an output of the reference waveform storage unit 107 and an output of the reception amplification unit 106; addition means 109 adds the outputs of the correlator 108; a display 111 displays an output of the addition means 109; and a synchronous timing generation unit TG controls the timings of the complementary code generator 110, the reference waveform storage unit 107 and the like, and the correlation between the output of the reception amplification unit 108 and the output of the reference waveform storage unit 107 is calculated by the correlator 108, and the calculated results are sequentially stored by using, for example, a shift register and the like, and also the waveform after a previous correlation process is outputted in synchronization with the waveform after the current correlation process.

As a result, the signal outputted by the addition means 109 becomes the signal whose range side lobe is improved (for example, refer to the following patent document 1).

Patent Document 1: Japanese Patent Application Publication after Examination 7-81993 (Page 6-7, FIG.9)

However, in the conventional ultrasonographic device, when such as a tissue of a living body, a test body is in motion, it is difficult to output the reception waveform after the correlation process based on a previous transmission, synchronously with the received signal after the correlation process based on a current transmission. As a result, there was a problem that the range side lobe of the signal outputted by the addition means was not improved.

DISCLOSURE OF THE INVENTION

The present invention is proposed in order to solve the conventional problems. Therefore, its object is to provide an ultrasonographic device that can improve the range side lobe of the complementary coded transmission and reception method even for the moving test body such as the living body.

The ultrasonographic device of the present invention comprises: transmission and reception means for carrying out transmission and reception of a modulated waveform signal obtained by modulating by a complementary code, and transmission and reception of a normal waveform signal that is not modulated; velocity detection means for detecting a velocity of a motion of an interest region of a test body from the received signal received by the transmission and reception means; and delay process means for changing a delay time of the modulated waveform signal that is received by the transmission and reception means correspondingly to the velocity detected by the velocity detection means.

This configuration reduces the range side lobe by using the complementary code transmission and reception method even if the test body is in motion.

Moreover, the ultrasonographic device of the present invention comprises: transmission and reception means for carrying out transmission and reception of a modulation waveform signal obtained by modulating by a complementary code, and transmission and reception of a normal waveform signal that is not modulated; velocity detection means for detecting a velocity of a motion of an interest region of a test body from a received signal received by the transmission and reception means; and changing means for changing the code length of the complementary code of the transmission and reception means correspondingly to the velocity detected by the velocity detection means. This configuration reduces the range side lobe by using the complementary code transmission and reception method even if the test body is in motion.

Moreover, the ultrasonographic device of the present invention comprises a configuration in which the velocity detection means detects the velocity of the motion of the interest region of the test body, by receiving the normal waveform signal from the transmission and reception means.

This configuration reduces the range side lobe by using the complementary code transmission and reception method even if the test body is in motion.

Moreover, in the ultrasonographic device of the present invention, the transmission and reception means comprises means for changing a reception sensibility in accordance with the code length of the complementary code of the modulation waveform signal to be transmitted and received. This configuration can decrease the reception sensibility and reduce the noise level when the length of the code becomes long. For example, when the length of the code is N, the reception sensibility may be decreased to 1/2N.

Moreover, in the ultrasonographic device of the present invention, the transmission and reception means comprises a configuration for changing a central frequency of an ultrasonic to be transmitted and received in accordance with the code length of the complementary code of the modulation waveform signal to be transmitted and received. With this configuration, when the code becomes longer, the sensibility becomes higher, which can make the central frequency higher and make a resolution higher.

Moreover, in the ultrasonographic device of the present invention, the velocity detection means comprises a function for detecting the dispersion of the velocities of the motions of the interest region. This configuration reduces the range side lobe by using the complementary code transmission and reception method even if the test body is in motion.

Moreover, the ultrasonographic device of the present invention has a configuration that further has comprises for adding information with regard to the code length of the complementary code that is transmitted and received by the transmission and reception means to a diagnostic picture and can check the length of the code when the picture is obtained from the picture information.

Moreover, the ultrasonographic device of the present invention has a configuration that further comprises display means for displaying information with regard to the code length of the complementary code that is transmitted and received by the transmission and reception means and can check the length of the code from the picture.

The present invention can provide the ultrasonographic device having the effect of reducing the range side lobe by using the complementary code transmission and reception method even if the test body is in motion, because as for the transmission and reception unit, the switching between the transmission and reception of the modulation waveform signal that is modulated with the complementary code and the transmission and reception of the normal waveform signal that is not modulated, or the change of the length of the complementary code is possible, and as for the means for detecting the velocity of the motion of the interest region of the test body, the switching between the transmission and reception of the modulation waveform signal that is modulated with the complementary code in accordance with the velocity and the transmission and reception of the normal waveform signal, or the change of the length of the complementary code is carried out.

BRIEF DESCRIPTION OF THE DRAWINGS

FIG. 1 is a block diagram of an ultrasonographic device according to a first embodiment of the present invention.
FIG. 2A is a view showing an output signal example of a correlator according to the first embodiment of the present invention.
FIG. 2B is a view showing an output signal example of the correlator according to the first embodiment of the present invention.
FIG. 2C is a view showing an output signal example of addition means according to the first embodiment of the present invention.
FIG. 3A is a view showing an output signal example of a transmission unit corresponding to a usual transmission and reception according to the first embodiment of the present invention.
FIG. 3B is a view showing an example of a complementary code corresponding to the usual transmission and reception according to the first embodiment of the present invention.
FIG. 3C is a view showing an output signal example of a transmission unit corresponding to transmission and reception of complementary codes according to the first embodiment of the present invention.
FIG. 4 is a detailed block diagram of a correlator 7 according to the first embodiment of the present invention.
FIG. 5 is a block diagram of an ultrasonographic device according to a second embodiment of the present invention.
FIG. 6 is a block diagram of an ultrasonographic device according to a third embodiment of the present invention.
FIG. 7 is the block diagram of the conventional ultrasonographic device.

BEST MODE FOR CARRYING OUT THE INVENTION

Hereinbelow, description will be given about the ultrasonographic device according to the embodiments of the present invention by using the drawings.

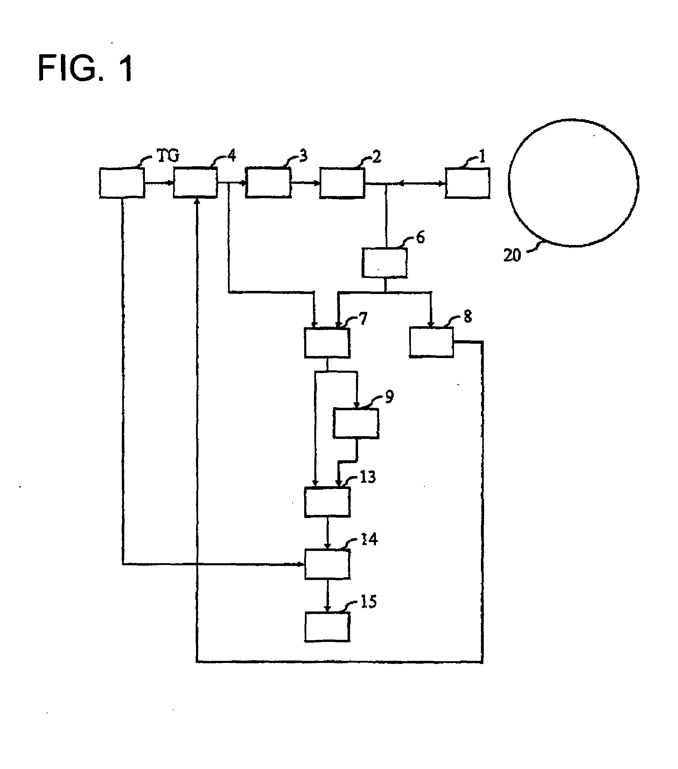

FIG. 1 shows the ultrasonographic device according to the first embodiment of the present invention.
In FIG. 1, an output of a coded waveform generation unit 3 is supplied to the transmission unit 2 for driving a prove 1, in accordance with an output of a complementary code generator 4, and a reception amplification unit 6 amplifies a received signal from the prove 1, and an output of the reception amplification unit 6 is inputted to velocity detection means 8, and an output of the velocity detection means 8 is inputted to the complementary code generator 4, and a correlator 7 performs a correlation calculation between an output of the complementary code generator 4 and an output of the reception amplification unit 6, and a memory 9 stores an output of the correlator 7, and addition means 13 adds the outputs of the correlator 7 and the memory 9, and an output of the addition means 13 is processed by a signal procession unit 14, and a display 15 displays an output of the signal procession unit 14, and a synchronous timing generation unit TG controls the timings of the complementary code generator 4 and the like.

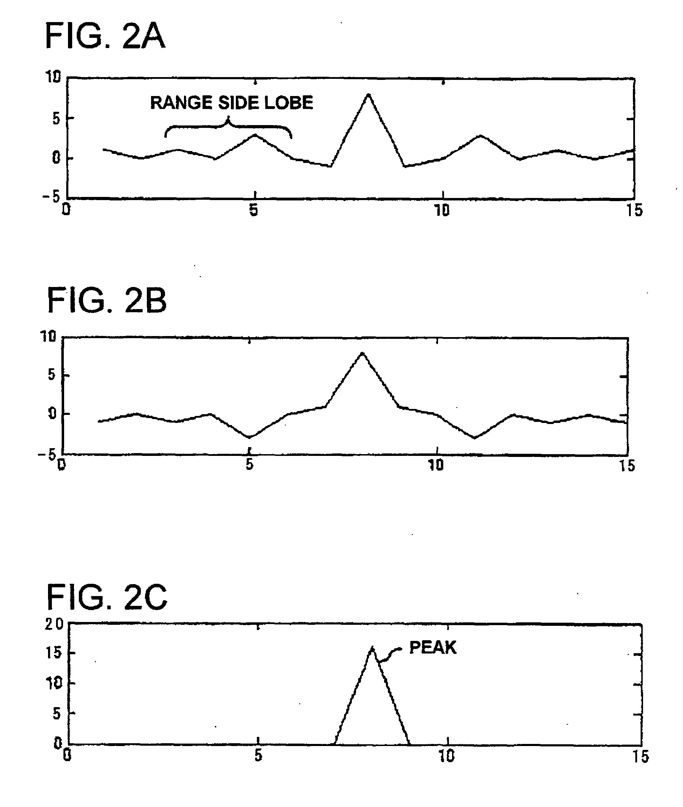

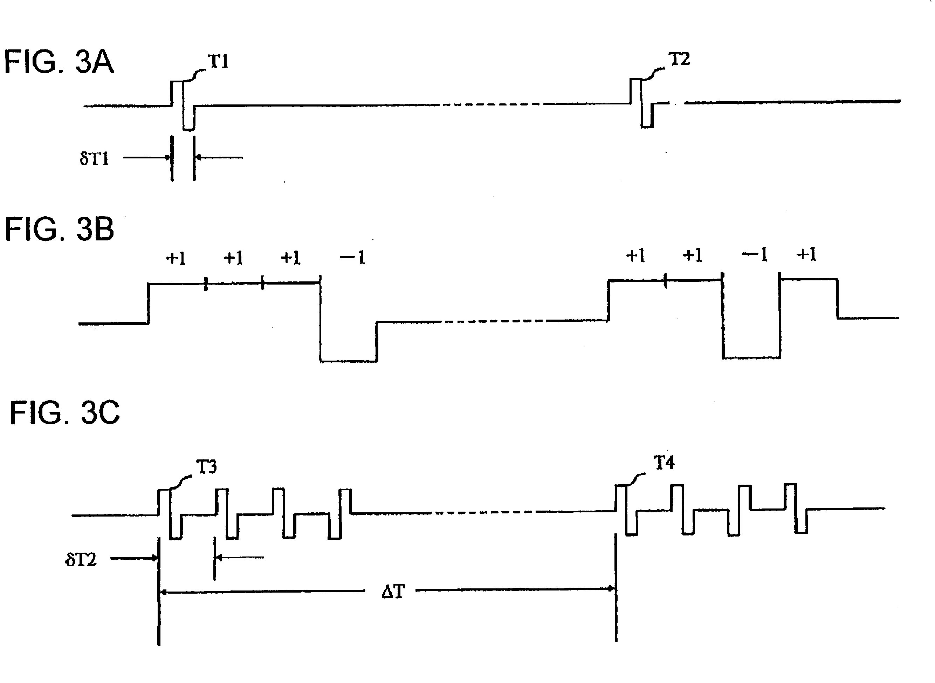

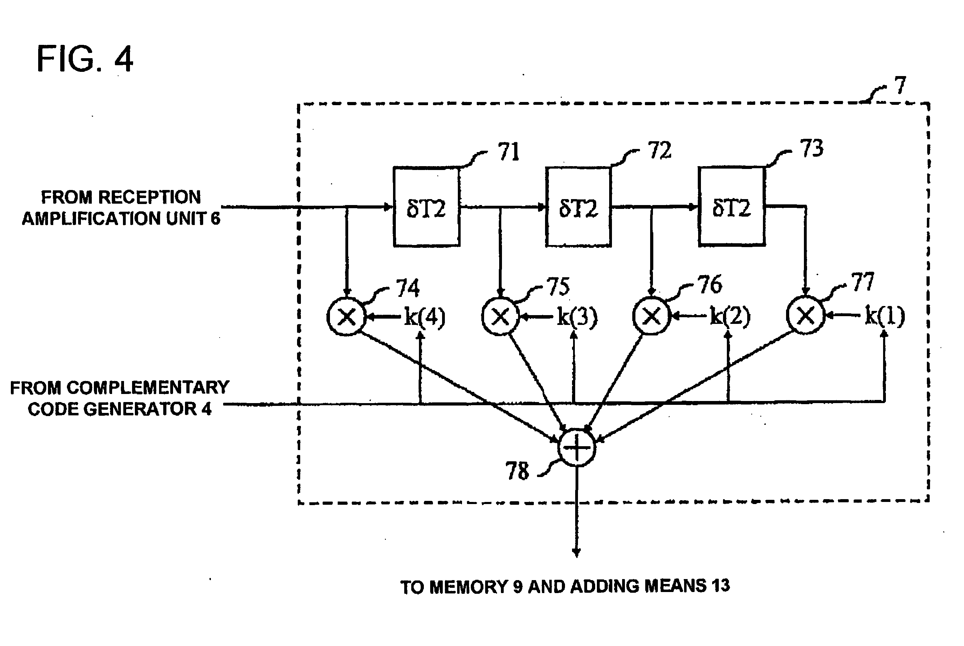

A test body 20 is brought into contact with the prove 1. FIG. 2A, FIG. 2B and FIG. 2C are the waveform views describing the principle of the complementary code transmission and reception, and FIG. 3A, FIG. 3B and FIG. 3C are the outputs of the transmission unit 2 in the usual transmission and reception and the coded transmission and reception. FIG. 4 is the detailed block diagram of the correlator 7, and the output of the reception amplification unit 6 is inputted to delay circuits 71, 72 and 73. The output of the reception amplification unit 6 is inputted to multipliers 74, 75, 76 and 77 and multiplied by complementary codes k(1), k(2), k(3) and k(4) generated by the complementary code generator 4 and added in an adder 78.

The operations of the above-configured ultrasonographic device will be described below by using FIG. 1, FIG. 2A, FIG. 2B, FIG. 2C, FIG. 3A, FIG. 3B, FIG. 3C and FIG. 4.

Firstly, the complementary code is described.

The complementary code has the following features. For example, a2 and b2 of the following equation are the complementary codes of a length N=2 m (m=1, represents a power).

$$a2 = [+1, +1] \tag{1}$$

$$b2 = [+1, -1] \tag{2}$$

an autocorrelation c2 of a2 and an autocorrelation d2 of b2 are represented by the following equation.

$$c2 = [+1, +2, +1] \tag{3}$$

$$d2 = [-1, +2, -1] \tag{4}$$

A sum e2 of the autocorrelation c2 and the autocorrelation d2 becomes:

$$e2 = [0, +4, 0] \tag{5}$$

Then, at the peak of the center of a sequence, the value becomes 2N=4, and before and after the peak, namely, the range side lobe becomes zero.

The complementary code of N=2 m (m=2) is obtained from a procedure that in accordance with the complementary code of the length N, an item in which b2 is linked after a2 is defined as a4, and an item in which the code of b2 is inverted and linked after a2 is defined as b4.

$$a4 = [+1, +1, +1, -1] \tag{6}$$

$$b4 = [+1, +1, -1, +1] \tag{7}$$

FIG. 2A and FIG. 2B are the views showing the autocorrelation of the complementary code of N=2 m (m=3), and FIG. 2C is the view showing the sum of the autocorrelations. Then, at the peak of the center of the sequence, the value is 2N=16, and before and after the peak, namely, the value of the range side lobe is zero.

In this way, when the length of the complementary code becomes N, the peak value of a received signal becomes 2N times. Thus, when the complementary code becomes long, for example, in the signal procession unit 14, a reception sensibility can be decreased, which can relatively reduce the noise level included in the received signal. For example, when the length of the complementary code is N, the reception sensibility may be set to 1/2N. Reversely, when the length of the complementary code becomes short, the reception sensibility may be increased.

Fig. 3A is the view showing an impulse that is the output waveform of the transmission unit 2 in the usual transmission and reception, FIG. 3B is the view showing an example of the complementary code outputted by the complementary code generator 4, and FIG. 3C is the view showing the output waveform of the transmission unit 2 that corresponds to the complementary code in FIG. 3B. The complementary code in FIG. 3B corresponds to the equations (6), (7). The pulse widths of impulses T1, T2 of the transmission unit 2 in the usual transmission and reception shown in FIG. 3A are $\delta$ T1. The output waveform of the transmission unit 2 that corresponds to the complementary code shown in FIG. 3C is the waveform equal to the impulse T1 when the value of the complementary code in FIG. 3B is +1, and when the value of the complementary code is -1, it is the waveform in which the impulse T1 is inverted.

In a waveform T3, the impulses T1 or the waveform-inverted impulses T1 are arranged at a $\delta$ T2 interval. By the way, there is a relation of $\delta$ T1 < $\delta$ T2. Also, an interval between the waveform T3 and a waveform T4 is $\Delta$T. As for the received signal through the waveform T3, by using the complementary code of the equation (6), the correlator 7 performs an autocorrelation process thereon, and as for the received signal through the waveform T4, by using the complementary code of the equation (7), the correlator 7 performs the autocorrelation process thereon. Then, after the output of the correlator 7 that corresponds to the waveform T3 is stored in the memory 9 and delayed by a time $\Delta$T and read out, this is added by the addition means 13. Consequently, a complementary transmission and reception output is obtained.

The process in the correlator 7 is specifically shown by using FIG. 4. The correlator 7 shown in FIG. 4 corresponds to the complementary code of the length N=4 of the equations (6), (7). The output of the reception amplification unit 6 is inputted to the delay circuits 71, 72 and 73, and the delay of the time $\delta$ T2 is given to each of them. The $\delta$ T2 is the interval of the impulses included in the output waveforms corresponding to the complementary codes in already-described FIG. 3C. The output of the amplification unit 6 is multiplied by the coefficients k(1), k(2), k(3) and k(4) in the multipliers 74, 75, 76 and 77. The coefficient k(j) (1≦j≦4) is the complementary code of the length N=4 of the equations (6), (7). When the output waveform of the transmission unit 2 corresponds to the complementary code of the equation (6), the coefficient k(j) corresponds to the equation (6), and when the output waveform of the transmission unit 2 corresponds to the complementary code of the equation (7), the coefficient k(j) corresponds to the equation (7). In the foregoing description, in order that at the output of the addition means 13, the range side lobe is zero as indicated by the equation (5), the test body 20 is required to be static.

However, when the test body 20 is a living body, its tissue is in motion. In particular, when the received signal from the tissue of a circulatory organ group is processed, it is required not to receive the influence of the pulsation of the tissue. The velocity operation means 8 performs a Doppler operation process on the output signal from the reception amplification unit 6 and detects a movement velocity V of the tissue.

When the Doppler operation process is carried out, the output waveform of the transmission unit 2 may use the impulse of the usual transmission and reception. At first, the complementary code generator 4 carries out the output corresponding to the usual transmission and reception. The coded waveform generation unit 3 generates the impulse waveform of FIG. 3A. The velocity operation means 8 measures the velocity for the interest region of the test body 20.

When the velocity detected by the velocity operation means 8 becomes equal to a constant level or less, the complementary code generator 4 generates the complementary code of the equation (6) at a certain time. Then, the output of the correlator 7 is stored in the memory 9. After the $\Delta$T time, the complementary code generator 4 generates the complementary code of the equation (7). Then, the output of the correlator 7 and the output read from the memory 9 are added by the addition means 13. While the complementary code transmission and reception is carried out, the velocity detection of the velocity operation means 8 is carried out through the usual transmission and reception at a proper temporal interval. When the detected velocity V becomes equal to or higher than the constant level, the transmission and reception through only the usual transmission and reception is carried out.

Or, on the basis of the velocity detected by the velocity operation means 8, the length of the complementary code may be changed. For example, as the velocity becomes faster, the complementary code may be shorter. Or, when the code of the complementary code becomes longer, the central frequency of the ultrasonic may be made higher. Moreover, as a result of the Doppler operation process, when the distribution of the velocities is spread even if the average velocity is equal to or less than the constant level, namely, when the velocities are dispersed, the complementary code generator 4 may be designed to generate the complementary code of the equation (6) at a certain time, when the dispersion becomes equal to or less than the constant level.

Moreover, the length information of the code of the complementary code may be added to the picture information obtained by the signal procession unit 14. Moreover, the length information of the code of the complementary code added to the picture information together with the picture of the test body may be displayed on the display 15.

According to the ultrasonographic device according to the first embodiment of the present invention as mentioned above, as for the transmission and reception unit, the switching between the transmission and reception of a modulation waveform signal modulated with the complementary code and the transmission and reception of the normal waveform signal that is not modulated is possible, and as for the means for detecting the velocity of the motion in the interest region of the test body, by carrying out the switching between the transmission and reception of the modulation waveform signal modulated with the complementary code correspondingly to the velocity and the transmission and reception of the normal waveform signal that is not modulated, the accurate complementary code transmission and reception can be carried out in which it is difficult to receive the influence of the motion of the test body.

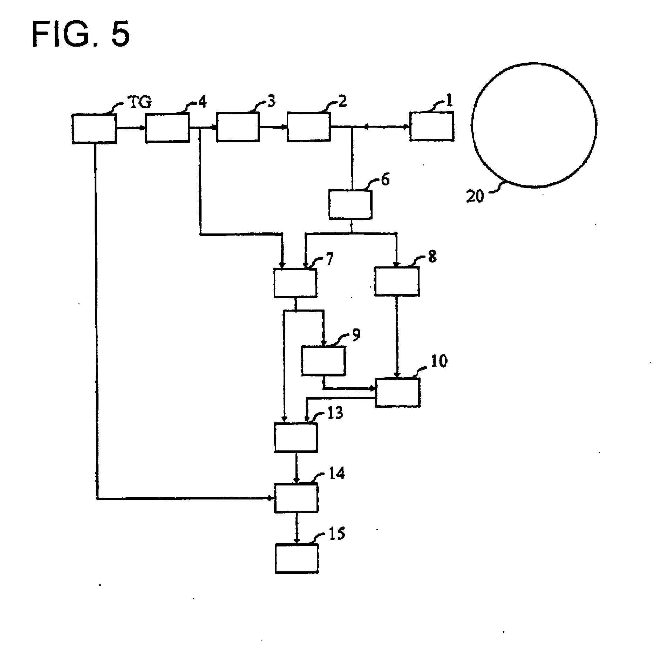

Next, the ultrasonographic device according to the second embodiment of the present invention is shown in FIG. 5.

In FIG. 5, for that having the same action and function as the first embodiment, its description is omitted. In FIG. 5, an output of the velocity detection means 8 is connected to delay means 10. An output of the memory 9 is inputted to the delay means 10, and an output of the delay means 10 is inputted to the addition means 13.

As for the ultrasonographic device configured as mention above, its operations will be described below by using FIG. 5.

At first, the velocity detection means 8 detects the velocity V of the motion of the interest region of the test body 20. The waveform of the coded waveform generation unit 3 in this case may be the waveform corresponding to the usual transmission and reception or may be the waveform corresponding to the coded transmission and reception. Since the motional velocity is V, after the $\Delta T$ time that is the interval between the transmissions, the position of the interest region is changed by $\Delta TL = V \cdot \Delta T$.

With the change of the position of $\Delta L$, an arrival time of an echo from the interest region is changed by $\Delta\Delta T = 2 \cdot \Delta L/c$ (however, c is a sound velocity inside the test body). For this reason, in the interest region, the temporal interval of the corresponding echo is $\Delta T - \Delta\Delta T$. In this way, in the delay means 10, the delay time of $\Delta\Delta T$ is adjusted for the signal outputted by the memory 9. Then, the output of the delay means 10 and the output of the correlator 7 are added by the addition means 13.

As mentioned above, according to the ultrasonographic device according to the second embodiment of the present invention, the output of the velocity detection means 8 is connected to the delay means 10. The output of the memory 9 is inputted to the delay means 10, and the output of the delay means 10 is inputted to the addition means 13. Thus, even if the interest region of the test body 20 is in motion, the accurate complementary code transmission and reception can be carried out in which it is difficult to receive the influence of the motion of the test body.

Next, the ultrasonographic device according to the third embodiment of the present invention is shown in FIG. 6.

In FIG. 5, for that having the same action and function as the first embodiment, its description is omitted. In the test body 20, an electrocardiograph 16 for detecting its pulsating is installed. Then, an output of the electrocardiograph 16 is inputted to R-wave trigger delay means 12, and an output of the R-wave delay means 12 is inputted to the complementary code generator 4.

For the ultrasonographic device configured as mentioned above, its operations will be described below by using FIG. 6.

At first, the prove 1 is assumed such that a carotid artery wall of the test body 20 serves as the interest region. On the other hand, the electrocardiograph 16 is assumed to observe a cardiac electrograph of a heart of the test body 20.

The contraction and dilation of the heart involves the change in the blood vessel diameter of the carotid artery. However, a timing when, since the carotid artery diameter is maximized or minimized, a carotid artery wall is instantaneously rested has a certain temporal delay from the contraction and dilation of the heart. For this reason, the R-wave trigger delay means 12 for delaying an R-wave trigger outputted by the electrocardiograph 16 estimates a time when the carotid artery diameter is maximized or minimized. Then, at a time when the carotid artery wall is instantaneously rested, the complementary code generator 4 generates the complementary code and carries out the complementary code transmission and reception.

As mentioned above, according to the ultrasonographic device according to the third embodiment of the present invention, the electrocardiograph 16 is installed inside the test body 20, the output of the electrocardiograph 16 is inputted to the R-wave delay means 12, and the output of the R-wave delay means 12 is inputted to the complementary code generator 4. Thus, even if the interest region of the test body 20 is in motion, the accurate complementary code transmission and reception can be carried out in which it is difficult to receive the influence of the motion of the test body.

By the way, in the foregoing descriptions, the prove 1 may be composed of the transducer of a single element or may be configured such that a plurality of transducers are arrayed.

INDUSTRIAL APPLICABILITY

As described above, in the ultrasonographic device according to the present invention, as for the transmission and reception unit, the switching between the transmission and reception of the modulation waveform signal modulated with the complementary code and the transmission and reception of the normal waveform signal that is not changed, or the change of the length of the complementary code is possible, and as for the means for detecting the velocity of the motion of the interest region of the test body, the switching between the transmission and reception of the modulation waveform signal modulated with the complementary code correspondingly to the velocity and the transmission and reception of the normal waveform signal, or the change of the length of the complementary code is carried out. Thus, there is an effect that, even if the test body is in motion, the complementary code transmission and reception method is used to reduce the range side lobe, and this is useful as the ultrasonographic device that uses the complementary coded transmission and reception method and the like.

**Claims**

1. An ultrasonographic device comprising:

   transmission and reception means (1) for carrying out transmission and reception of a modulated waveform signal obtained by modulating by a complementary code, and
   transmission and reception of a normal waveform signal;
   velocity detection means (8) for detecting a velocity of a motion of an interest region of a test body from a received signal received by said transmission and reception means (1);
   **characterised in** the normal waveform signal being not modulated and by further comprising
   delay process means (71, 72, 73) for changing a delay time of said modulated waveform signal that is received by said transmission and reception means (1) correspondingly to the velocity detected by said velocity detection means (8).

2. An ultrasonographic device comprising:

   transmission and reception means (1) for carrying out transmission and reception of a modulated waveform signal obtained by modulating by a complementary code, and
   transmission and reception of a normal waveform signal;
   velocity detection means (8) for detecting a velocity of a motion of an interest region of a test body from a received signal received by said transmission and reception means (1);
   **characterised in** the normal waveform being not modulated and by further comprising
   changing means for changing the code length of the complementary code of said transmission and reception means correspondingly to the velocity detected by said velocity detection means.

3. The ultrasonographic device according to any one of claims 1 and 2, wherein said velocity detection means (8) is adapted to detect the velocity of the motion of the interest region of the test body, by receiving said normal waveform signal from said transmission and reception means (1).

4. The ultrasonographic device according to any one of claims 1 and 2, herein said transmission and reception means (1) is adapted to change a reception sensibility in accordance with the code length of the complementary code of said modulation waveform signal to be transmitted and received.

5. The ultrasonographic device according to any one of claims 1 and 2, wherein said transmission and reception means (1) is adapted to change a central frequency of ultrasonic waves to be transmitted and received in accordance with the code length of the complementary code of said modulated waveform signal to be transmitted and received.

6. The ultrasonographic device according to any one of claims 1 and 2, wherein said velocity detection means (8) has a function for detecting the dispersion of the velocities of the motions of the interest region.

7. The ultrasonographic device according to any one of claims 1 and 2, further comprising means for adding information with regard to the code length of the complementary code that is transmitted and received by said transmission and reception means to a diagnostic picture.

8. The ultrasonographic device according to any one of claims 1 and 2, further comprising display means (15) for displaying information with regard to the code length of the complementary code that is transmitted and received by said transmission and reception means (1).

**EP 2 133 026 B1**

Patentansprüche

1.  Ultraschallgerät, umfassen:

    Sende- und Empfangsmittel (1) zum Ausführen der Sendung und des Empfangs eines modulierten Wellenformsignals, das durch Modulieren eines Komplementärcodes erzielt ist, und der Sendung und des Empfangs eines normalen Wellenformsignals;
    Geschwindigkeitserkennungsmittel (8) zum Erkennen einer Bewegungsgeschwindigkeit eines Bereichs von Interesse eines Testkörpers aus einem empfangenen Signal, das durch das Sende- und Empfangsmittel (1) empfangen ist;
    **dadurch gekennzeichnet, dass** das normale Wellenformsignal nicht moduliert ist, und dass es ferner Folgendes umfasst:

    Verzögerungsprozessmittel (71, 72, 73) zum Ändern einer Verzögerungszeit des modulierten Wellenformsignals, das durch das Sende- und Empfangsmittel (1) empfangen ist, entsprechend der Geschwindigkeit, die durch das Geschwindigkeitserkennungsmittel (8) erkannt ist.

2.  Ultraschallgerät, umfassend:

    Sende- und Empfangsmittel (1) zum Ausführen der Sendung und des Empfangs eines modulierten Wellenformsignals, das durch Modulieren eines Komplementärcodes erzielt ist, und der Sendung und des Empfangs eines normalen Wellenformsignals;
    Geschwindigkeitserkennungsmittel (8) zum Erkennen einer Bewegungsgeschwindigkeit eines Bereichs von Interesse
    eines Testkörpers aus einem empfangenen Signal, das durch das Sende- und Empfangsmittel (1) empfangen ist;
    **dadurch gekennzeichnet, dass** das normale Wellenformsignal nicht moduliert ist, und dass es ferner Folgendes umfasst:

    Änderungsmittel zum Ändern der Codelänge des Komplementärcodes des Sende- und Empfangsmittels entsprechend der Geschwindigkeit, die durch das Geschwindigkeitserkennungsmittel erkannt ist.

3.  Ultraschallgerät nach einem der Ansprüche 1 oder 2, wobei das Geschwindigkeitserkennungsmittel (8) dazu geeignet ist, die Bewegungsgeschwindigkeit des Bereichs von Interesse des Testkörpers durch Empfangen des normalen Wellenformsignals von dem Sende- und Empfangsmittel (1) zu erkennen.

4.  Ultraschallgerät nach einem der Ansprüche 1 oder 2, wobei das Sende- und Empfangsmittel (1) dazu geeignet ist, eine Empfangsempfindlichkeit gemäß der Codelänge des Komplementärcodes des Modulationswellenformsignals, das gesendet und empfangen werden soll, zu ändern.

5.  Ultraschallgerät nach einem der Ansprüche 1 oder 2, wobei das Sende- und Empfangsmittel (1) dazu geeignet ist, eine zentrale Frequenz von Ultraschallwellen, die gesendet und empfangen werden sollen, gemäß der Codelänge des Komplementärcodes des modulierten Wellenformsignals, das gesendet und empfangen werden soll, zu ändern.

6.  Ultraschallgerät nach einem der Ansprüche 1 oder 2, wobei das Geschwindigkeitserkennungsmittel (8) eine Funktion zum Erkennen der Dispersion der Bewegungsgeschwindigkeiten des Bereichs von Interesse aufweist.

7.  Ultraschallgerät nach einem der Ansprüche 1 oder 2, ferner umfassend Mittel zum Hinzufügen von Information hinsichtlich der Codelänge des Komplementärcodes, der durch das Sende- und Empfangsmittel gesendet und empfangen ist, zu einem diagnostischen Bild.

8.  Ultraschallgerät nach einem der Ansprüche 1 oder 2, ferner umfassend Anzeigemittel (15) zum Anzeigen von Information hinsichtlich der codelänge des Komplementärcodes, der durch das Sende- und Empfangsmittel (1) gesendet und empfangen ist.

**Revendications**

1.  Dispositif échographique comprenant :

un moyen (1) d'émission et de réception pour effectuer l'émission et la réception d'un signal de forme d'onde modulé obtenu par modulation par un code complémentaire, et l'émission et la réception d'un signal de forme d'onde normal ;

un moyen (8) de détection de vitesse pour détecter une vitesse d'un mouvement d'une zone d'intérêt d'un corps d'essai à partir d'un signal reçu par ledit moyen (1) d'émission et de réception ;

**caractérisé en ce que** le signal de forme d'onde normal n'est pas modulé et par le fait de comprendre en outre un moyen (71, 72, 73) de traitement de retard pour changer un temps de retard dudit signal de forme d'onde modulé qui est reçu par ledit moyen (1) d'émission et de réception de manière correspondante à la vitesse détectée par ledit moyen (8) de détection de vitesse.

2. Dispositif échographique comprenant :

un moyen (1) d'émission et de réception pour effectuer l'émission et la réception d'un signal de forme d'onde modulé obtenu par modulation par un code complémentaire, et l'émission et la réception d'un signal de forme d'onde normal ;

un moyen (8) de détection de vitesse pour détecter une vitesse d'un mouvement d'une zone d'intérêt d'un corps d'essai à partir d'un signal reçu par ledit moyen (1) d'émission et de réception ;

**caractérisé en ce que** le signal de forme d'onde normal n'est pas modulé et par le fait de comprendre en outre un moyen de changement pour changer la longueur du code complémentaire dudit moyen d'émission et de réception de manière correspondante à la vitesse détectée par ledit moyen de détection de vitesse.

3. Dispositif échographique selon l'une quelconque des revendications 1 et 2, dans lequel ledit moyen (8) de détection de vitesse est adapté pour détecter la vitesse du mouvement de la zone d'intérêt du corps d'essai, en recevant ledit signal de forme d'onde normal à partir dudit moyen (1) d'émission et de réception.

4. Dispositif échographique selon l'une quelconque des revendications 1 et 2, dans lequel ledit moyen (1) d'émission et de réception est adapté pour changer une sensibilité de réception conformément à la longueur du code complémentaire dudit signal de forme d'onde de modulation à émettre et à recevoir.

5. Dispositif échographique selon l'une quelconque des revendications 1 et 2, dans lequel ledit moyen (1) d'émission et de réception est adapté pour changer une fréquence centrale d'ondes ultrasonores à émettre et à recevoir conformément à la longueur du code complémentaire dudit signal de forme d'onde modulé à émettre et à recevoir.

6. Dispositif échographique selon l'une quelconque des revendications 1 et 2, dans lequel ledit moyen (8) de détection de vitesse a une fonction de détection de la dispersion des vitesses des mouvements de la zone d'intérêt.

7. Dispositif échographique selon l'une quelconque des revendications 1 et 2, comprenant en outre un moyen pour ajouter des informations concernant la longueur du code complémentaire qui est émis et à reçu par ledit moyen d'émission et de réception à une image de diagnostic.

8. Dispositif échographique selon l'une quelconque des revendications 1 et 2, comprenant en outre un moyen (15) d'affichage pour afficher des informations concernant la longueur du code complémentaire qui est émis et reçu par ledit moyen (1) d'émission et de réception.

# FIG. 1

# FIG. 2A

RANGE SIDE LOBE

# FIG. 2B

# FIG. 2C

PEAK

## FIG. 3A

T1

T2

δT1

## FIG. 3B

+1   +1   +1   −1         +1   +1   −1   +1

## FIG. 3C

T3

T4

δT2

ΔT

# FIG. 4

FROM RECEPTION
AMPLIFICATION UNIT 6

FROM COMPLEMENTARY
CODE GENERATOR 4

TO MEMORY 9 AND ADDING MEANS 13

EP 2 133 026 B1

# FIG. 5

# FIG. 6

# FIG. 7    PRIOR ART

**EP 2 133 026 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005111131 A
- JP 7081993 A